# EUROPEAN PATENT APPLICATION

(11) **EP 1 820 455 A1**
(43) Date of publication of application: **22.08.2007**
(21) Application number: 04822531.2
(22) Date of filing: 07.12.2004
(51) Int. Cl.: A61B 17/04, A61M 37/00, B05B 7/08, B05B 7/24

(54) **BIOPOLYMER POWDER GELATING/JETTING APPARATUS**

(71) Applicant: Nakanishi Inc., Kanuma-shi, Tochigi 322-8666 (JP)
(72) Inventor: Kawata, Sousaku, Nakanishi Inc., Tochigi, 3228666 (JP)
(74) Representative: Heyer, Volker
(86) International application number: PCT/JP2004/018195
(87) International publication number: WO 2006/061883

(57) **Abstract**

A system is provided for efficiently gelating and injecting a constant amount of biopolymer powder for effectively using biopolymer in sealing, stanching, and prevention of adhesion of a post-surgical site. Powder transfer line 320 for transferring biopolymer powder atomized with the pressure of noninflammable gas in powder agitating container 220, and transfer lines 310, 330 for separately transferring noninflammable gas and solution separately from the powder transfer line 320, are joined at the tip of nozzle attachment 400 for injecting a mixture of the biopolymer powder and the noninflammable gas, together with the noninflammable gas and the solution.

## Description

The present invention relates to a system for gelating and injecting biopolymer powder, which is used for sealing, stanching, or preventing adhesion of surgical sites after surgery, such as laparotomy, laparoscopic surgery, or endoscopic surgery.

Biocompatible biopolymers, such as oxidized cellulose, carboxymethyl cellulose, hyaluronic acid, and collagen, have conventionally been used by applying to surgical sites during surgery or to wound sites for the purpose of hemostasis, prevention of adhesion, prevention of keloid, wound treatment, or close-up or sealing of cuts. Such biopolymers are usually in the form of fiber sheets, films, granules, or gels. However, the sheet or the like form prevents application of biopolymers for hemostasis or prevention of adhesion in a body cavity or to a post-surgical site of endoscopic surgery due to lack of enough space.

In light of this, technology has been developed that allows precise attachment and arrangement of a biopolymer irrespective of the size, shape, and location of the application site, and is disclosed, for example, in Patent Publication 1. Patent Publication 1 discloses that a biopolymer is made into fluidized fine particles under the injection force of a gas, which particles are sprayed with a gas injecting agent (noninflammable gas) onto a post-surgical site in a body cavity or of endoscopic surgery. Patent Publication 1: JP-2003-62057-A

However, the invention disclosed in this publication is directed to sprayable fine biopolymer particles for hemostasis or prevention of adhesion, 80% of which are in the particle size range of up to 100 µm in the particle size distribution, which has the average particle size of not larger than 50 µm, which may be fluidized with a gas, andwhichmaybe used for hemostasis, prevention of adhesion, prevention of keloid, wound treatment, close-up or sealing precisely at an application site, irrespective of the size, shape, and location of the application site. No spraying system for practical application of the fine particles has been found. Incidentally, there is a conventional product which adopts a technology for making biopolymer powder into a sprayable form. In this apparatus, a console box containing biopolymer powder is placed on a separate vibrator for stirring, which is extremely complex in structure and expensive.

It is therefore an object of the present invention to provide a system for gelating and injecting biopolymer powder which is capable of gelating and injecting a constant amount of biopolymer powder for effective use in sealing, stanching, or preventing adhesion of a post-surgical site.

For achieving the above object, the system for gelating and injecting biopolymer powder according to the present invention is characterized in that it comprises a gas supplier for supplying noninflammable gas; a gas transfer line connected to said gas supplier for transferring said noninflammable gas; a powder agitating container connected to said gas supplier for agitating and atomizing biopolymer powder with gas pressure of said noninflammable gas supplied from said gas supplier; a powder transfer line connected to said powder agitating container for transferring said biopolymer powder; a solution supplier for supplying a solution for gelating said biopolymer; a solution transfer line connected to said solution supplier for transferring said solution; a nozzle attachment having a powder transfer channel which is connected to the powder transfer line and encloses a gas transfer channel connected to the gas transfer line and a solution transfer channel connected to the solution transfer line, for injecting the biopolymer powder with the noninflammable gas and the solution; a controller for controlling the gas supplier and the solution supplier; and an operating switch connected to said controller for switching ON/OFF the operations of said gas supplier and the solution supplier,
wherein said system gelates and injects the biopolymer powder by operation of the operating switch.

The present invention may be embodied as follows. First, the operating switch may be configured to be mounted on or near the nozzle attachment or at the operator's site including his hand. In this case, it is preferred that the controller has a gas pressure detecting means for detecting gas pressure in the signal gas supply line supplied with signal gas, and converts signals depending on the change in gas pressure in the signal gas supply line to control operation of the gas supplier and the solution supplier. It is also preferred that the operating switch is connected to the signal gas supply line via a signal gas transfer line extending near to the operator's site for transferring the signal gas, and has an actuating valve for opening/closing the signal gas transfer line and a push button for opening/closing the actuating valve, and switches ON/OFF the operation of the gas supplier and the solution supplier by means of pressing operation of the push button. Second, the powder transfer line is made of a conductive tube. Third, the gas supplier, the solution supplier, and the controller are provided in a console box, and connections of one-touch locking type, each allowing connection of the gas transfer line and the powder agitating container to the gas supplier, the solution transfer line to the solution supplier, or the operating switch to the controller, are exposed on the console box. Fourth, in the nozzle attachment, the gas transfer channel and the solution transfer channel are inserted into and extend in parallel and in contact with each other through the powder transfer channel. The tips of the gas transfer channel and the solution transfer channel project from the tip of the powder transfer channel. The nozzle attachment has a mechanism for generating a whirl at the tips of the gas transfer channel and the solution transfer channel. Fifth, the controller is composed of a microcomputer, and has a control function to start the gas supplier at a low pressure and to gradually increase the gas pressure to a predetermined level.

The biopolymer as used herein means one or more biocompatible polymers having hemostatic and anti-adhesion properties, such as carboxymethyl cellulose, carboxyethyl cellulose, oxidized cellulose, chitin, chitosan, hyaluronic acid, starch, glycogen, alginates, pectin, dextran, chondroitin sulfate, gelatin, and collagen. The noninflammable gas to be mixed with the polymer for transfer may be carbon dioxide gas, nitrogen gas, or the like, and the solution for gelating the transferred polymer may be saline or the like.

With the above structure, the system for gelating and injecting biopolymer powder according to the present invention provides remarkable effect of efficiently gelating and injecting a constant amount of biopolymer powder for effective use of the biopolymer in sealing, stanching, and preventing adhesion of post-surgical sites.
Fig. 1 is a plan view illustrating the overall structure of the system for gelating and injecting biopolymer powder according to the present invention.
Fig. 2 is a plan diagram showing various devices inside the console box of the present system.
Fig. 3 is a view showing various operation parts on the back face of the console box of the present system.
Fig. 4 is a view showing various operation parts on the front face of the console box of the present system.
Fig. 5 is a view showing various operation parts on the side face of the console box of the present system.
Fig. 6 is a front view of the operating switch of the present system.
Fig. 7 is a side view of the nozzle attachment of the present system.
Fig. 8 is a view showing the principal part of the nozzle attachment of the present system.
Fig. 9 is a view showing the principal part of the nozzle attachment of the present system.

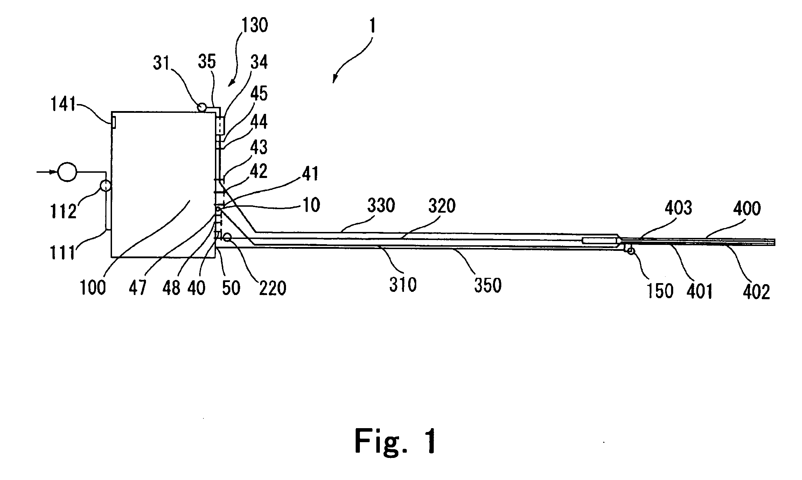

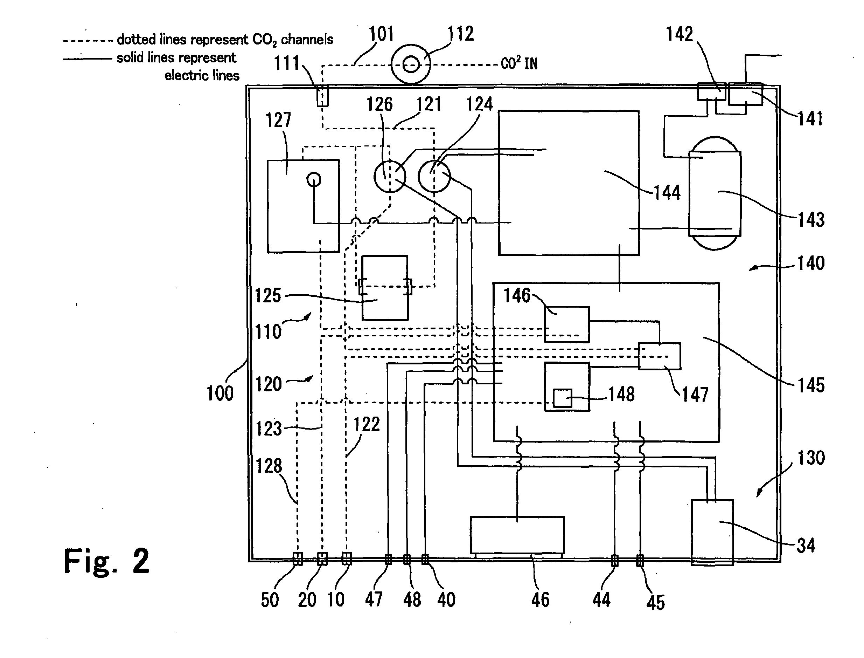

The system for gelating and injecting biopolymer powder according to the present invention (simply referred to as gel injection system 1 hereinbelow) will now be explained with reference to the attached drawings. Referring to Figs. 1 and 2, the gel injection system 1 includes gas supplier 110 for supplying noninflammable gas; gas transfer line 310 connected to the gas supplier 110 for transferring the noninflammable gas; powder agitating container 220 connected to the gas supplier 110 for agitating and atomizing biopolymer powder with gas pressure of the noninflammable gas supplied from the gas supplier 110; powder transfer line 320 connected to the powder agitating container 220 for transferring the biopolymer powder; solution supplier 130 for supplying a solution for gelating the biopolymer powder; solution transfer line 330 connected to the solution supplier 130 for transferring the solution; nozzle attachment 400 having powder transfer pipe (powder transfer channel) 402, which is connected to the powder transfer line 320 and encloses gas transfer pipe (gas transfer channel) 401 connected to the gas transfer line 310 and solution transfer pipe (solution transfer channel) 403 connected to the solution transfer line 330, for injecting the biopolymer powder with the noninflammable gas and the solution; controller 140 for controlling the gas supplier 110 and the solution supplier 130; and operating switch 150 connected to the controller 140 for switching ON/OFF the operations of the gas supplier 110 and the solution supplier 130. The overall gel injecting system 1 may be divided generally into four sections, i.e., the first section including the gas supplier 110, the solution supplier 130, the controller 140, and the operating switch 150; the second section including the powder agitating container 220; the third section including the gas transfer line 310, the powder transfer line 320, and the solution transfer line 330; and the fourth section including the nozzle attachment 440. Each section will now be explained below.

Referring to Figs. 1 and 2, the gas supplier 110, the solution supplier 130, and the controller 140 of the first section are assembled in console box 110. On the surface of the console box 100 are exposed and arranged connections for connecting the gas transfer line 310 and the powder agitating container 220 to the gas supplier 110, a connection for connecting the solution transfer line 330 to the solution supplier 130, and a connection for connecting the operating switch 150 to the controller 140, and also various operation parts are provided. The operating switch 150 is structured to extend near to the operator's site for remote control.

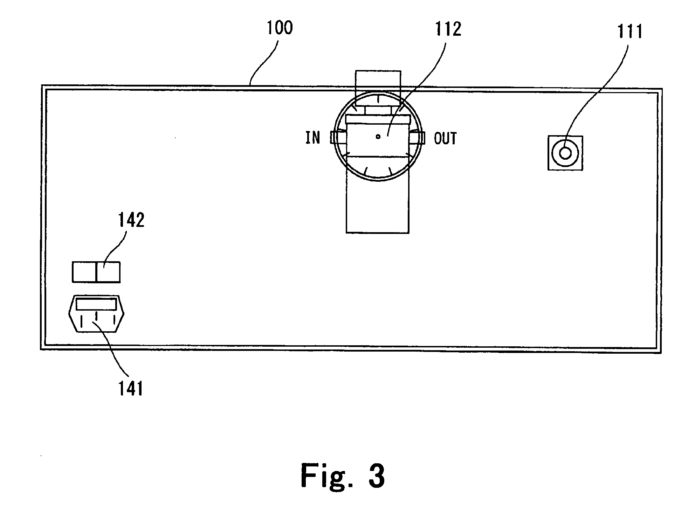

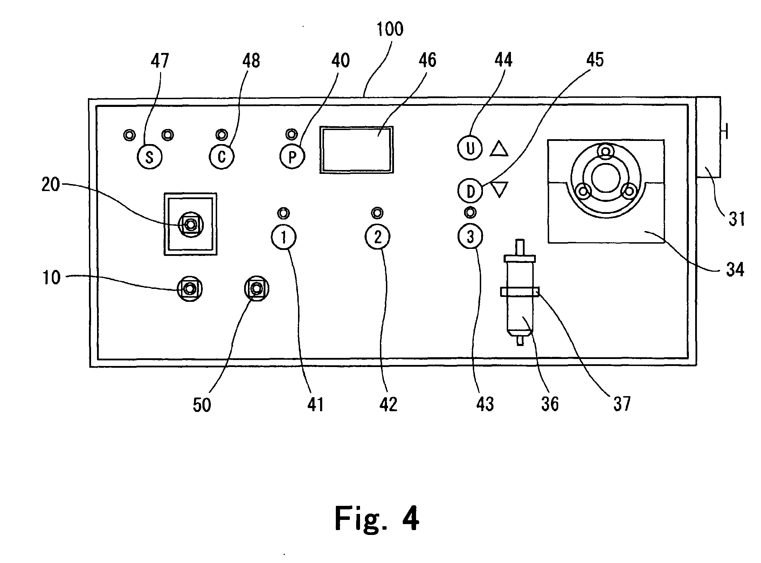

The gas supplier 110 is composed of gas pressure regulator 112 connected to a source of noninflammable gas, and gas source connection socket 111, both provided on the back face of the console box 100 as shown in Fig. 3; gas transfer line connection socket 10 to which the gas transfer line 310 is connected, agitating container connection socket 20 to which the powder agitating container 220 is connected, signal gas connection socket 50 to which signal gas transfer line 350 is connected, all arranged on the front face of the console box adjacent to each other as shown in Fig. 4; gas supply line 120 connecting the gas source connection socket 111 to the gas transfer line connection socket 10 and the agitating container connection socket 20 for providing a gas channel for supplying the noninflammable gas, and incidental devices arranged in the gas supply line 120, such as solenoid valves 124, 126 and electronic pressure regulator 127, and signal gas supply line 128 connected to the signal gas connection socket 50 for supplying, as a signal gas, noninflammable gas at the same pressure as that of the noninflammable gas supplied to the gas supply line 120, all arranged inside the console box 100 as shown in Fig. 2. In this case, as shown in Fig. 2, carbon dioxide gas cylinder (carbon dioxide gas) is used as a source of noninflammable gas, and external gas line 101 of the carbon dioxide gas cylinder is connected via gas pressure regulator 112 to the gas source connection socket 111. The gas supply line 120 is composed of a plurality of pipe sections. That is, the gas supply line is composed of incoming gas supply line 121 extending from the gas source connection socket 111, the incoming gas supply line 121 being branched at the other end into two directions, first outgoing gas supply line 122 connected to one of the branches of the incoming gas supply line 121 and extending to the gas transfer line connection socket 10, and second outgoing gas supply line 123 connected to the other of the branches of the incoming gas supply line 121 and extending to the agitating container connection socket 20. In the middle of the incoming gas supply line 121, first solenoid valve 124 for opening/closing the gas line and gas filter 125 (biofilter) for filtering the gas are provided in this order from the gas source connection socket 111. In the middle of the first outgoing gas supply line 122 is disposed second solenoid valve 126 for opening/closing the gas channel under the control of the controller 140 to be discussed later, and pressure sensor 147 also controlled by the controller 140 is connected downstream of the valve 126. In the middle of the second outgoing gas supply line 123 is disposed electronic pressure regulator 127 for regulating the gas flow (flow rate) under the control of the controller 140 to be discussed later, and electronic operation substrate 146 also controlled by the controller 140 is operatively connected downstream of the regulator 127. The signal gas supply line 128 is connected to the pressure sensor 147, from which is supplied the noninflammable gas at the same pressure as that of the noninflammable gas supplied to the gas supply line 120. For each of the connection sockets 111, 10, 20, 30, 50 (gas source connection socket 111, gas transfer line connection socket 10, agitating container connection socket 20, and signal gas connection socket 50), a gas socket of one-touch locking type is employed, wherein, by inserting each external line (external gas line 101, gas transfer line 310, powder agitating container 320, and signal gas transfer line 350) into each connection socket, a lock key provided on the terminal end of one of the connection socket and the external line engages in a key way provided on the other.

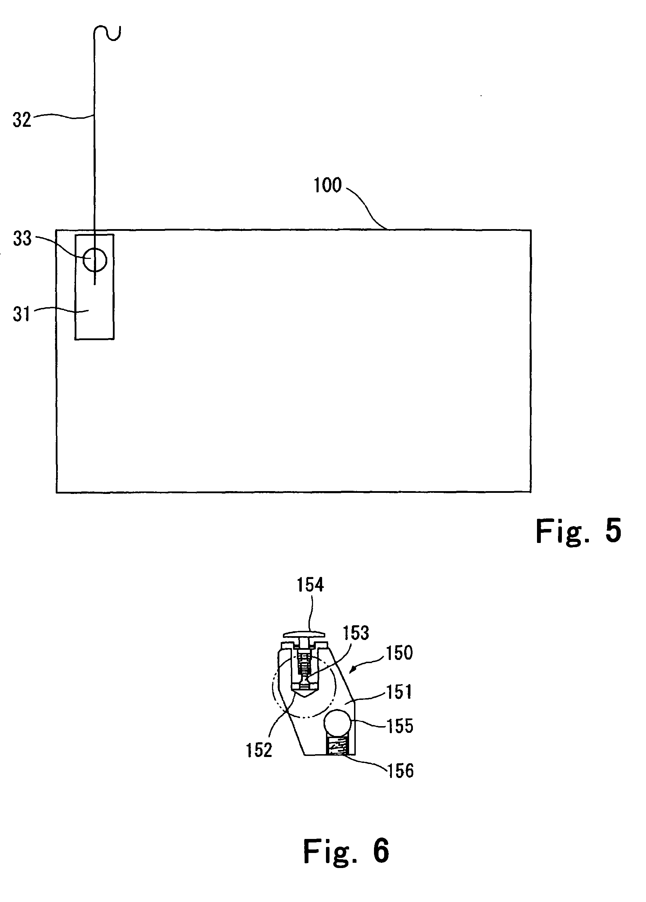

Referring to Fig. 1, the solution supplier 130 is composed of hanger stand 31 disposed outside the console box 100 for hanging a solution bottle containing the solution, solution supply pump (pump motor) 34 disposed inside the console box 100 for supplying the solution, and solution transfer line 35 connecting the solution bottle and the solution supply pump 34. In this case, saline is used as the solution. The hanger stand 34 is attached on the side face of the console box 100 as shown in Fig. 5. In this figure, 32 refers to a hanger pole and 33 refers to a screw for fixing the hanger pole. Referring to Fig. 2, the pump motor 34 is positioned in the upper front part of the interior of the console box 5, and its connection to the solution transfer line 330 is exposed on the front face of the console box 100 as shown in Fig. 4. This connection also employs a one-touch connector. This pump motor 34 is operatively connected to the first and second solenoid valves 124, 126 of the gas supplier 110 inside the console box 100 as shown in Fig. 2, and controlled by the controller 140. Incidentally, in Fig. 4, 36 refers to a saline reservoir chamber hanger and 37 refers to a chamber.

Referring to Fig. 2, the controller 140 is composed of a microcomputer arranged inside the console box 100, and includes control board 144 for controlling the apparatus 110, 130, and control memory board 145 connected to the control board 144 and storing predetermined operation programs. On the back face of the console box 100 are provided power input socket 141 for inputting power, and power ON/OFF switch 142 for switching ON/OFF the power. Power transformer 143 is disposed in the console box 100, and connected to the power input socket 141 via the power ON/OFF switch 142. To the power transformer 143 is connected the control board 144, to which the first and second solenoid valves 124, 126 of the gas supplier 110 and the electronic pressure regulator 127 are connected. In this embodiment of the controller 140, the control memory board 145 is particularly provided with electronic operation substrate 146, pressure sensor 147, and gas switch sensor 148. The electronic operation substrate 146 is operatively connected to the second outgoing gas supply line 123 of the gas supplier 110, and functions to introduce the noninflammable gas via the agitating container connection socket 20 into the powder agitating container 220. The pressure sensor 147 is connected to the first outgoing gas supply line 122, and functions to introduce gas of the same pressure as the gas supplied to the first outgoing gas supply line 122 into the signal gas supply line 128 (and then into the signal gas transfer line 350) and to detect the gas pressure in the signal gas supply line 128 (and the signal gas transfer line 350) . The control memory board 145 stores three operation modes for controlling the electronic pressure regulator 127. The three operation modes include a sealing mode wherein the gas flow rate from the electronic pressure regulator 127 is regulated for injecting amounts of the biopolymer powder and the noninflammable gas suitable for sealing; a hemostatic mode wherein the gas flow rate is regulated suitably for hemostasis; and an adhesion mode wherein the gas flow rate is regulated suitably for prevention of adhesion. At the beginning of each operation mode, the gas flow rate is adjusted so that the gas supplier 110 is started at a low pressure, and the gas pressure is gradually increased to a predetermined level under the control of the microcomputer. In order to set these operation modes, as shown in Figs. 2 and 4, sealing mode key 41, hemostasis mode key 42, adhesion mode key 43, and mode setting key 40, all connected to the control memory board 145, are provided on the front face of the console box 100, and a display lamp is also provided above each of the keys 41, 42, 43, and 40 for indicating the operation state of each key. Also on the front face of the console box 100, there are provided output up key 44 and output down key 45 for increasing or decreasing the output, respectively, at each operation mode, a digital display board 46 for indicating various information required for the operation, stand-by key 47, polymer refill/container change key 48, and display lamps therefor, which are all connected to the control memory board 145. With the power source ON, when the mode setting key 40 is pressed, the controller 140 becomes ready for setting an operation mode, and in this state when a desired mode key is pressed, the corresponding desired operation mode is set. From this state, by switching ON the operating switch 150 to be discussed later, ON-control is performed wherein the gas pressure in the signal gas supply line 128 is decreased, which is detected by the pressure sensor 147, and the signal is converted in accordance with the control program stored in the control memory board 145, and the control signals are transmitted from the control board 144 to the first and second solenoid valves 124, 126 and the electronic pressure regulator 127 to open the first and second solenoid valves 124, 126 and to operate the electronic pressure regulator 127 in each operation mode, in cooperation with which the pump motor 34 is driven. On the other hand, by switching OFF the operating switch 150, OFF-control is performed wherein the gas pressure in the signal gas supply line 128 is made constant, which is detected by the pressure sensor 147, and the first and second solenoid valves 124, 126 are closed and the electronic pressure regulator 127 is stopped, in cooperation with which the pump motor 34 is stopped. In the system of the present invention, the supply rate of the biopolymer powder and the solution (saline) is controlled for each operation mode by increasing or decreasing from the standard ratio of 7: 3 by means of the microcomputer in the console box 100.

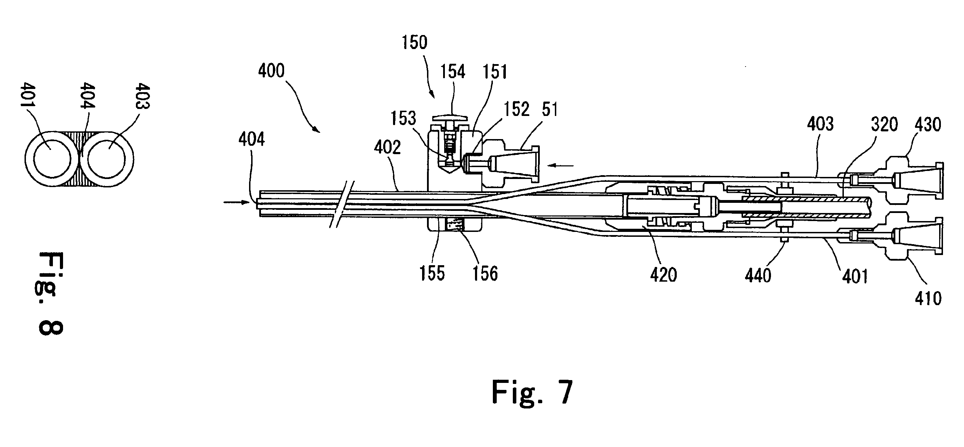

The controller 140 employs a control system wherein the operation of the gas supplier 110 and the solution supplier 130 is controlled by the signal from the sensor which detects the gas pressure in the signal gas supply line 128 supplied with the signal gas, and converts the signal depending on the change in gas pressure in the signal gas supply line 128. Thus, as shown in Figs. 6 and 7, as the operating switch 150, a switch having a valve mechanism is employed, which includes actuating valve 153 connected to the signal gas transfer line 350 for opening/closing the same, which is in turn connected to the signal gas supply line 128 in the console box 100, and push button 154 for opening/closing the actuating valve 153. Here, the operating switch 150 is formed of small block structure 151, and includes gas inlet port 152, into which socket for connecting signal gas transfer line is inserted for connecting the signal gas transfer line 350, a gas outlet port (not shown) for discharging the gas to outside, the actuating valve 153 for opening/closing the gas outlet port to open/close the signal gas transfer line 350, and the push button 154 for opening/closing the actuating valve 153. In this embodiment, the actuating valve 153 is opened by pressing down the push button 154. By this opening operation of the actuating valve 153, the noninflammable gas in the signal gas transfer line 350 is discharged to outside to reduce the gas pressure in the signal gas transfer line 350 and the signal gas supply line 128 the console box 100, to thereby switching ON the operation of the gas supplier 110 and the solution supplier 130. The operating switch 150 also includes, as shown in Fig. 7, mounting portion 155 and fixing member 156 adapted to the shape of the member to which the switch 150 is to be attached, so that the switch 150 may be mounted on or near the nozzle attachment 400 to be discussed later or to the operator's site including his hand. In this embodiment, the mounting portion 155 of the operating switch 150 is formed through the block structure 151 as a through hole, through which the nozzle attachment 400 may be inserted. The fixing member 156 is a screw, which is screwed from the outer surface of the operating switch 150 into the through hole to fasten the nozzle attachment 400. By tightening/loosening the screw, the operating switch 150 may be mounted on the nozzle attachment 400 at a desired location. The mounting portion 155 of the operating switch 150 may be modified arbitrarily depending on the shape of the member to which the switch 150 is to be attached, and may be in the form of, for example, a ring or a belt (or a band). In this way, the signal gas transfer line 350 extending to the operator's site is connected (at its leading end) to the signal gas connection socket 50 of the console box 100, and (at its terminal end) to the socket 51 for connecting signal gas supply line fixedly connected to the gas inlet port 152 of the operating switch 150.

Referring to Fig. 1, the powder agitating container 220 in the second section has a container body for accommodating the biopolymer powder, inlet connection port for connecting to the agitating container connection socket 20, and outlet connection port to which the powder transfer line 320 may be connected. In the powder agitating container 220, the gas flow line extending from the inlet connection port branches into bifurcate gas lines, each having at its tip a nozzle having a built-in duckbill check valve for discharging powder agitating gas (simply referred to as a check valve hereinbelow), through which nozzle the gas is distributed to five outlets, i.e., upper, lower, right, and left outlets arranged at 90° intervals and an outlet in the direction of outlet from the check valve (straight direction), in total of ten outlets for two check valves, for injection into the container body. In the container body, a transfer line for a mixture of the noninflammable gas and the biopolymer powder is provided, with the inlet end for the mixture being arranged in the upper portion of the container body and the outlet end being arranged at the outlet connection port. The interior of the powder agitating container 220 is lined with a non-electrostatic resin for preventing electrostatic charge of the mixture.

Referring to Fig. 1, the gas transfer line 310, the powder transfer line 320, the solution transfer line 330, and the signal gas transfer line 350 in the third section are made of synthetic resin tubes so as to be disposable (for single use), and made flexible and bendable. In particular, the powder transfer line 320 is formed of a conductive tube of a selected conductive material having excellent property to remove electrostatic charge of the mixture.

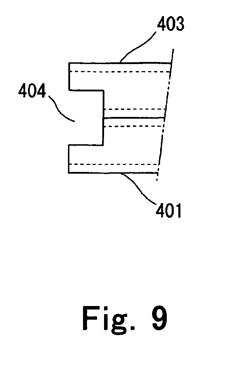

Still referring to Fig. 1, the nozzle attachment 400 in the fourth section has powder transfer pipe 402 for injecting the biopolymer powder with the noninflammable gas, gas transfer pipe 401 for injecting the noninflammable gas, and solution transfer pipe 403 for injecting the solution. As shown in Fig. 7, the powder transfer pipe 402 is a thin needle-like tube made of stainless steel (SUS316). On the base end of the powder transfer pipe 402, tube connector 420 of a one-touch pocket-fit type is attached for fixedly connecting the powder transfer pipe 402 and the powder transfer line 320. The gas transfer pipe 401 and the solution transfer pipe 403 are in the form of still thinner tubes insertable into the powder transfer pipe 402, and are similarly made of stainless steel (SUS316). On the base end of the gas transfer pipe 401, tube connector 410 of a one-touch operation type is attached for fixedly connecting the gas transfer pipe 401 and the gas transfer line 310. Similarly, on the base end of the solution transfer pipe 403, tube connector 430 of a one-touch operation type is attached for fixedly connecting the solution transfer pipe 403 and the solution transfer line 330. The gas transfer pipe 401 and the solution transfer pipe 403 are arranged outside of and symmetrically with respect to the powder transfer pipe 402, and fixed by means of clamp member 440 to the powder transfer line 320 connected to the powder transfer pipe 402. In the middle of the powder transfer pipe 402, the gas transfer pipe 401 and the solution transfer pipe 403 are symmetrically inserted from outside into the powder transfer pipe 402, and extend therethrough in parallel and in contact with each other toward the tip end, so as to be arranged as a gas supply channel and a solution supply channel in the powder transfer pipe 402. The tips of the gas supply pipe (gas supply channel) 401 and the solution supply pipe (solution supply channel) 403 slightly project from the tip of the powder transfer pipe 402 to provide whirl generating mechanism 404 for generating whirl flow at the tips of the gas transfer pipe 401 and the solution transfer pipe 403. Here, the whirl generating mechanism 404 is formed by cutting off the inner semicircular halves of the tips of the gas transfer pipe 401 and the solution transfer pipe 403 in the form of a dent, as shown in Figs. 8 and 9.

Next, the use, operating procedure, and operating state of the gel injection system 1 will now be explained with selective reference to Figs. 1 and 9. As shown in Figs. 2 and 3, a power cord is connected to the power input socket 141 on the back face of the console box 100 for supplying power. Next, the external gas line 101 from a gas source is connected to the gas source connection socket 111. Here, the gas pressure is adjusted to a predetermined level by means of the gas pressure regulator 112, where necessary.

As shown in Figs. 1 and 5, the solution bottle, in this case, a saline pack, is hung on the hanger stand 31 on the side face of the console box 100, and the solution bottle and the solution supply pump 34 are connected with the solution transfer line 35.

Next, as shown in Figs. 1 and 4, the solution transfer line 330 is connected to the solution supply pump 34 on the front face of the console box 100. Then a filter is set in the gas transfer line connection socket 10, and the gas transfer line 310 is connected thereto. A filter is also set in the agitating container connection socket 20, and the powder agitating container 220 is connected thereto. The powder transfer line 320 is connected to the outlet connection port of the powder agitating container 220.

As shown in Fig. 7, the powder transfer line 320 is connected to the tube connector 420 of the pocket-fit type of the nozzle attachment 400. The gas transfer line 310 is connected to the tube connector 410 of the nozzle attachment 400. The solution transfer line 330 is connected to the tube connector 430 of the nozzle attachment 400.

As shown in Fig. 4, the signal gas transfer line 350 is connected to the signal gas connection socket 50 on the front face of the console box 100. The signal gas transfer line 350 is also connected to the socket 51 for connecting signal gas supply line of the operating switch 150 attached to the nozzle attachment 400, as shown in Fig. 7. In this way, the set up of the gel injection system 1 is completed.

Next, the biopolymer powder is placed in the powder agitating container 220 inside the console box 100. The power ON/OFF switch 142 on the back face of the console box 100 (shown in Fig. 3) is switched ON, and the stand-by key 47 on the front face of the console box 100 (shown in Fig. 4) is pressed to stand-by. The mode setting key 40 is pressed, and one of the mode keys 41, 42, and 43 is selected and pressed, depending on the application of the gel injection system 1. All the preparation required before use of the gel injection system 1 is now complete.

After this, the nozzle attachment 400 will be operated. This operation is performed by an operator grasping the nozzle attachment 400, directing the tip of the nozzle attachment 400 toward the application site of a patient, and operating the operating switch 150 at his hand. When the operator presses with his finger the push button 154 of the operating switch 150 at hand, the gas supplier 110 and the solution supplier 130 are started under the control of the controller 140 in the console box 100.

In the gas supplier 110 (shown in Fig. 2), the first and second solenoid valves 124, 126 are opened, and the electronic pressure regulator 127 is operated according to the selected operation mode. The noninflammable gas in the gas source, i.e., carbon dioxide gas (simply referred to as gas hereinbelow), is introduced into the gas supply line 120 at a predetermined pressure. Here, in the second outgoing gas supply line 123, the flow (flow rate) of the gas is regulated by means of the electronic pressure regulator 127, whereby the gas is started to be supplied at a low pressure, and the pressure is gradually increased to a predetermined level. The gas through the first outgoing gas supply line 122 is introduced into the gas transfer line 310 via the gas transfer line connection socket 10, and transferred toward the nozzle attachment 400. The gas through the second outgoing gas supply line 123 is introduced into the powder agitating container 220 via the agitating container connection socket 20 to agitate and atomize the biopolymer powder in the powder agitating container 220 at a predetermined pressure. The mixture of the gas and the biopolymer powder is introduced into the powder transfer line 320, through which the mixture is transferred toward the nozzle attachment 400. The gas at the same pressure as that in the gas supply line 120 is introduced into the signal gas transfer line 350.

In the solution supplier 130 (shown in Fig. 2), the pump motor 34 is driven, by which the solution in the solution bottle, i. e. saline, is transferred to the solution transfer line 330, and then to the nozzle attachment 400 in parallel with the noninflammable gas in the gas transfer line 310.

In the nozzle attachment 400 (shown in Fig. 7), the gas from the gas transfer line 310 is transferred to the gas transfer pipe 401 via the tube connector 410, while the solution from the solution transfer line 330 is transferred to the solution transfer pipe 403 via the tube connector 430, whereby the gas and the solution are injected through the tips of the gas transfer pipe 401 and the solution transfer pipe 403. Here, by means of the whirl generating mechanism 404 at the tips of the gas transfer pipe 401 and the solution transfer pipe 403, the gas and the solution are injected in a whirl flow. On the other hand, the mixture of the gas and the biopolymer powder from the powder transfer line 320 is transferred to the powder transfer pipe 402 via the tube connector 420, and at the tip of the powder transfer pipe 402, the mixture is joined together with the whirl flow of the gas and the solution, and gelated and injected. Here, when the selected operation mode is the sealing mode, the amount of the biopolymer powder and the hardness/softness of the gel are adjusted to be suitable for sealing, before the injection of the gel. Similarly, when the selected operation mode is the hemostatic mode, the amount of the biopolymer powder and the hardness/softness of the gel are adjusted to be suitable for hemostasis, before the injection of the gel. When the selected operation mode is the anti-adhesion mode, the amount of the biopolymer powder and the hardness/softness of the gel are adjusted to be suitable for prevention of adhesion, before the injection of the gel. As mentioned above, the injection rate of the biopolymer powder and the solution (saline) is increased or decreased from the standard ratio of 7:3.

When the operator releases his finger from the push button 154 of the operating switch 150 at hand, the operation of the gas supplier 110 and the solution supplier 130 are stopped under the control of the controller 140 in the console box 100, so that the injections of the noninflammable gas, the solution, and the mixture of the noninflammable gas and the biopolymer powder are stopped.

According to this embodiment, the powder transfer line 320 transferring the biopolymer powder that has been atomized by the pressure of the noninflammable gas in the powder agitating container 220, and the separate supply lines 310, 330 for the noninflammable gas and the solution, respectively, separate from the powder transfer line 320, are joined at the tip of the nozzle attachment 400 to inject the mixture of the biopolymer powder and the noninflammable gas together with the noninflammable gas and the solution. Thus, a constant amount of biopolymer powder from the powder agitating container 220 may be effectively gelated and injected, while clogging is prevented to the end up to the nozzle attachment 400. Therefore, with the gel injection system 1, the biopolymer may effectively be used for sealing, hemostasis, and prevention of adhesion.

According to the embodiment particularly discussed above, the nozzle attachment 400 is composed of the powder transfer pipe 402, and the gas transfer pipe 401 and the solution transfer pipe 403 extending through the powder transfer pipe 402, and the gas transfer pipe 401 and the solution transfer pipe 403 are extended in parallel and in contact with each other inside the powder transfer pipe 402, and the tips of the gas transfer pipe 401 and the solution transfer pipe 403 are projected from the tip of the powder transfer pipe 402. Thus, the mixture of the biopolymer powder and the noninflammable gas may be prevented from scattering at the treatment site to the utmost. Here, whirl flow is generated by denting the inner semicircular halves of the tips of the gas transfer pipe 401 and the solution transfer pipe 403, so that the gelation of the biopolymer powder may still be promoted, responding to the change in the control conditions of the solution and the biopolymer by the microcomputer.

According to the embodiment particularly discussed above, since the operating switch 150 is disposed at the hand of an operator (practitioner), operation at hand is advantageous in the operation site where a number of foot pedals for various instruments are scattered on the floor, and wrongly taking another instrument for the present system may be prevented. The operation of the present system 1 is switched ON/OFF by changing the pressure of the noninflammable gas by means of the pressing operation of the operating switch 150, so that the present system may be operated safely and securely.

According to the embodiment particularly discussed above, electrostatic charge-removing structure is employed in the powder agitating container 220 and the powder transfer line 320, so that even when the biopolymer powder is charged during agitation in the powder agitating container 220, the electrostatic charge may be removed from the powder agitating container 220 and the powder transfer line 320 to allow uniform dispersion and uniform injection of the biopolymer. Further, even when the nozzle attachment 400 is brought into contact with another object, no spark is generated, so that the present system 1 may be handled safely at a medical site.

According to the embodiment particularly discussed above, the connection sockets of one-touch locking type, each allowing connection of the gas transfer line 310 and the powder agitating container 220 to the gas supplier 110, the solution transfer line 330 to the solution supplier 130, or the operating switch 150 to the controller 140, are exposed on the console box 100. Thus, connection of the gas transfer line 310 and the powder agitating container 220 to the gas supplier 110, the solution transfer line 330 to the solution supplier 130, and the operating switch 150 to the controller 140, are facilitated.

According to the embodiment particularly discussed above, the controller 140 is constituted of a microcomputer, and the supply of gas to the powder agitating container 220 is started at a low pressure, and the pressure is gradually increased to a predetermined level under the control of the microcomputer. Thus a suitable amount of the biopolymer powder may be transferred from the powder agitating container 220 to the nozzle attachment 400. This copes with the drawbacks that, when the constant pressure of gas is introduced into the powder agitating container 220 from the start, the flow channel for constant injection of the biopolymer powder in the powder agitating container 220 is not ready, resulting in a more than adequate amount of the biopolymer powder. In this way, when the pressure of the gas to be sent to the powder agitating container 220 is set for each operation mode at the gas supplier 110, the amount of the biopolymer powder and the hardness/softness of the gel may suitably be adjusted for each operation mode.

In addition, according to the embodiment discussed above, since the lines 310, 320, and 330 in the third section are made disposable, in-hospital infection may be prevented. Further, since the lines 310, 320, and 330 are made flexible, operationality of the nozzle attachment 400 and the operating switch 150 for the operator may be improved. Since the nozzle attachment 400 in the fourth section is made of stainless steel (SUS316), the nozzle attachment maybe sterilized by steam and used repeatedly. The present system 1 in its entirety may be structured more simply than a conventional system, so that the system may be provided at a lower price compared to the conventional one.

Endoscopic surgery is rapidly becoming popular in the recent operation scene. Instrument is demanded for efficiently gelating and injecting a biomaterial for sealing, stanching, and preventing adhesion of the surgery site with a biomaterial. The present invention provides an efficient gel injection system at a relatively low cost. The present system is also effective in sealing, stanching, and preventing adhesion of a post-laparotomy site. The system of gelating a biomaterial and injecting the same to a surgery site is technically advantageous compared to application of a sheet onto a surgery site, which is currently in practice. Thus the present invention exhibits medical effects that have never been achieved.

## Claims

1. A system for gelating and injecting biopolymer powder comprising:
a gas supplier for supplying noninflammable gas;
a gas transfer line connected to said gas supplier for transferring said noninflammable gas;
a powder agitating container connected to said gas supplier for agitating and atomizing biopolymer powder with gas pressure of said noninflammable gas supplied from said gas supplier;
a powder transfer line connected to said powder agitating container for transferring said biopolymer powder;
a solution supplier for supplying a solution for gelating said biopolymer powder;
a solution transfer line connected to said solution supplier for transferring said solution;
a nozzle attachment having a powder transfer channel which is connected to the powder transfer line and encloses a gas transfer channel connected to the gas transfer line and a solution transfer channel connected to the solution transfer line, , for injecting the biopolymer powder with the noninflammable gas and the solution;
a controller for controlling the gas supplier and the solution supplier; and
an operating switch connected to said controller for switching ON/OFF the operations of said gas supplier and the solution supplier,
wherein said system gelates and injects the biopolymer powder by operation of the operating switch.

2. The system for gelating and injecting biopolymer powder according to claim 1, wherein said operating switch is configured to be mounted on or near the nozzle attachment or at an operator's site including his hand.

3. The system for gelating and injecting biopolymer powder according to claim 1 or 2, wherein said controller has a gas pressure detecting means for detecting gas pressure in the signal gas supply line supplied with signal gas, and converts signals depending on change in gas pressure in said signal gas supply line to control operation of said gas supplier and said solution supplier,
wherein said operating switch is connected to said signal gas supply line via a signal gas transfer line extending near to an operator's site for transferring the signal gas, and has an actuating valve for opening/closing said signal gas transfer line and a push button for opening/closing said actuating valve, and switches ON/OFF operation of said gas supplier and said solution supplier by means of pressing operation of said push button.

4. The system for gelating and injecting biopolymer powder according to any one of claims 1 to 3, wherein said powder transfer line is made of a conductive tube.

5. The system for gelating and injecting biopolymer powder according to any one of claims 1 to 4, wherein said gas supplier, said solution supplier, and said controller are provided in a console box, and connections of one-touch locking type each allowing connection of the gas transfer line and the powder agitating container to the gas supplier, the solution transfer line to the solution supplier, or the operating switch to the controller, are exposed on said console box.

6. The system for gelating and injecting biopolymer powder according to any one of claims 1 to 5, wherein said nozzle attachment includes the gas transfer line and the solution transfer line inserted into and extending in parallel and in contact with each other through the powder transfer line,
wherein tips of said gas transfer line and the solution transfer line project from a tip of said powder transfer line,
wherein said nozzle attachment has a mechanism for generating a whirl flow at tips of the gas transfer line and the solution transfer line.

7. The system for gelating and injecting biopolymer powder according to any one of claims 1 to 6, wherein said controller is composed of a microcomputer, and has a control function to start the gas supplier at a low pressure and to gradually increase a gas pressure to a predetermined level.
